# EUROPEAN PATENT SPECIFICATION

(11) **EP 0 516 751 B1**
(45) Date of publication and mention of the grant of the patent: **05.07.1995**
(21) Application number: 91905973.3
(22) Date of filing: 25.02.1991
(51) Int. Cl.: A61C 9/00

(54) **SCRIM-LINED THERMOPLASTIC DENTAL IMPRESSION TRAY**
MIT EINER VERSTÄRKUNGSEINLAGE VERSEHENER DENTALABDRUCKLÖFFEL
PORTE-EMPREINTE EN MATIERE THERMOPLASTIQUE DOUBLE DE TISSU

(30) Priority: 23.02.1990 US 484695
(43) Date of publication of application: 09.12.1992
(73) Proprietor: MINNESOTA MINING AND MANUFACTURING COMPANY, St. Paul, Minnesota 55133-3427 (US)
(72) Inventor: BRYAN, Thomas, T., Saint Paul, MN 55133 (US); UBEL, F., Andrew, III, Saint Paul, MN 55133 (US); OXMAN, Joel, D., Saint Paul, MN 55133 (US)
(74) Representative: Kristiansen, Alf P.
(86) International application number: PCT/US91/01244
(87) International publication number: WO 91/12775

(56) References cited:
- EP-A- 0 096 020
- FR-A- 2 374 889
- FR-B- 2 078 675
- US-A- 4 227 877

## Description

### CROSS-REFERENCE TO RELATED APPLICATION

This application is a continuation-in-part of U.S. Patent Application Serial No. 07/484,695, filed February 23, 1990.

### FIELD OF THE INVENTION

This invention relates to dental impression-taking using a customizable thermoplastic tray.

### BACKGROUND OF THE INVENTION

High molecular weight poly (epsilon-caprolactone) (also known as "polycaprolactone") has been used as a thermoplastic molding compound for general-purpose modelmaking and dentistry. References describing polycaprolactone molding compositions include U.S. Pat. No. 4,835,203; Kokai (Japanese Published Pat. Appl.) Nos. Sho 63[1988]-171554, Sho 63[1988]-270759; and TONE^{R} POLYMERS P-300 AND P-700 High Molecular Weight Caprolactone Polymers (1988 product literature of Union Carbide Corp.).

European Pat. Applicaton No. 0 359 135 was not published until 21 March 1990. It describes a mouthpiece (mouthguard) for use in contact sports. The mouthpiece is made of inner and outer layers of thermoplastic material. This reference mentions providing a rough inner surface on the outer layer, to improve adhesion to the inner layer material. No suggestion is made of any scrim lining.

A rigid plastic tray with a flocked interior is sold by Laclede under the name "Fuzzy Tray". Several manufacturers sell plastic or metal trays having perforations or projections that lock a cured impression material to the tray.

Japanese Published Pat. Application No. Sho 63[1988]-47706 describes a fiber reinforced acrylic plastic impression tray. Although not intended to be heat-softened during use, the tray is said to be reinforced with various materials including fibers and cloth. The cloth apparently would be placed within the tray (like the fibrous fillers actually exemplified in the reference) rather than on its surface.

### OTHER REFERENCES

Kokai Nos. Hei 2[1990]-60642, and Hei 2[1990]-60672 were not published until 1 March 1990. They refer respectively to a thermoplastic impression tray and a mouthguard, each containing a "core body which is either inserted or laminated as needed". The core body is said to serve as a support body preventing dripping of resins. The core body is said to be made from woven cloth made of fibers, or a nonwoven cloth, or a plastic net. A "co-extrusion method" is said to be used to insert or laminate the core body. Although no drawings of the resulting articles are included, the core body apparently lies within the article and is surrounded by thermoplastic resin.

### SUMMARY OF THE INVENTION

Some impression materials adhere poorly to thermoplastic tray materials, especially trays made of polycaprolactone. Perforating such a tray might improve the bond between the cured impression and the tray, but could undesirably weaken or distort the tray. Flocking the interior of the tray might also improve the bond between the cured impression and the tray, but at some added expense and inconvenience during manufacturing since flocking can be difficult to carry out on a commercial scale. Typically, a dentist uses a plain tray which the dentist or assistant lines with a thin coating of solvent-based adhesive. The tray adhesive must be permitted to dry for a few minutes before the impression material is added. If the tray adhesive is not thoroughly dried, the bond between impression material and tray can fail when the completed impression is pulled away from the teeth. This often leads to tearing or distortion of the impression material. Even if used properly, the tray adhesive is both time-consuming and environmentally undesirable.

US-A-4 227 877 (& FR-A-2 374 889) discloses a moldable impression tray comprising a thermoplastic material formed of an acrylic material, such as a copolymer of methylmethacrylate and monomethylmethacrylate, having a glass transition temperature T_{g} of between 100°F (37.8°C) and 135°F (57.2°C), preferably about 105°F (40.5°C). To resist displacement of the final impression material, the tray of said document is provided with chemical or mechanical retention devices, e.g. in the form of small spherical balls raised above the surface of the tray.

EP-A-0 096 020 describes an impression tray of plastics, the inside of which is provided with thread-shaped attachment means to form anchoring means when impression material is put onto the tray.

It is an object of the present invention to provide a scrim-lined plastic dental impression tray which permits impressioning without the need for a tray adhesive and which minimizes waste of the impression material.

This object is achieved according to the present invention by a dental impression tray according to claim 1.

The invention also provides a method for preparing an impression tray according to claim 10.

Preferred embodiments of the present invention are detailed in the respective dependent claims.

### BRIEF DESCRIPTION OF THE DRAWING

FIG. 1 is an overhead view of a shell of thermoplastic material from which trays of the invention can be prepared; and
FIGS. 2, 3, and 4 are perspective views of three full-arch trays of the invention.

### DETAILED DESCRIPTION

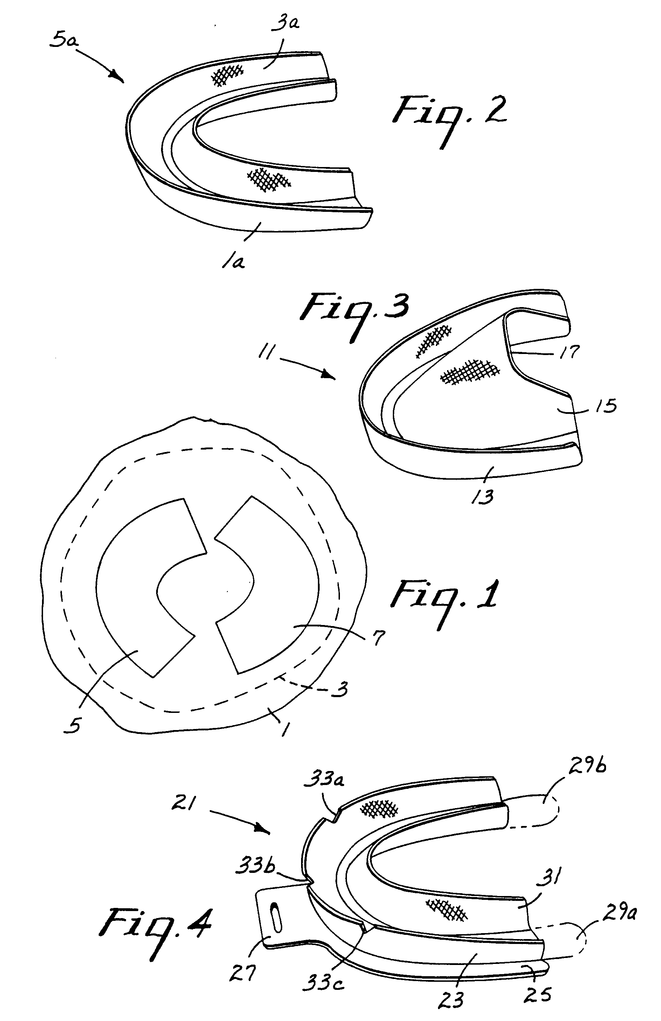

FIG. 1 shows a shell of thermoplastic material 1 atop a circular scrim patch 3, shown in phantom. The thermoplastic material has been warmed and pressed against the scrim patch, then permitted to cool, so that the thermoplastic material and scrim patch adhere strongly to one another. Custom tray blanks 5 and 7 have been formed using a handheld knife-edged stamping device (not shown) resembling a cookie cutter.

Referring to Fig. 2, arch-shaped tray 5a has been formed by heating the tray blank until the thermoplastic layer 1a softened, then placing scrim-covered surface 3a against a suitable U-shaped mandrel (not shown). The tray was permitted to cool until thermoplastic material 1a resolidified, then removed from the mandrel. The generally U-shaped trough in tray 5a conforms generally to the shape of the mandibular arch. The tray can be trimmed with a scissors, and reshaped as desired by heating and bending. If warmed, pressed against a patient's teeth and gingival tissue, and then cooled, it will form a close-fitting custom dental impression tray. The interior of the tray can be coated with a conventional dental impression material (e.g., a polyether or an addition-cured silicone), reinserted in the mouth, and once again pressed against the patient's teeth and gingiva until the impression material solidifies. Upon removal, the scrim 3a promotes a firm bond between the tray 5a and the hardened impression material, thereby discouraging tearing and facilitating the making of accurate impressions with minimal waste of impression material.

Fig. 3 illustrates a form of the invention particularly suited to impressions of the maxillary arch. Tray 11 includes thermoplastic material 13 and scrim 15. Palatal bridge 17 can be shaped to conform closely to the roof of the mouth. Bridge 17 stiffens tray 11, and facilitates the making of more accurate impressions.

Fig. 4 illustrates a form of the invention particularly suited to impressions involving undercuts or other shapes that frustrate removal of a conventional hardened impression. Tray 21 includes thermoplastic layers 23, 25. Layer 25 extends outward from the base of the tray and provides a ledge that facilitates tray removal. Tab 27 provides a handle that facilitates tray insertion and positioning during impressioning. Tray extensions 29a, 29b (shown in phantom) have been removed with a scissors. If desired, they can be left in place for impressions of long arches, or folded up to close off the open ends of tray 21, thereby minimizing spillage of the unhardened impression material. Scrim 31 promotes a strong bond between tray 21 and the hardened impression material (not shown). Notches 33a, 33b, 33c provide relief for the corresponding frenal membranes.

If desired, the trays of the invention can be less than a full arch (e.g., a quadrant). Also, the tray can have upper and lower open channels, so that the user can take a "double bite" impression of teeth in occlusion.

A variety of thermoplastic materials can be used to make the trays of the invention. Selection of the thermoplastic material should be based in part on the desired end use for the tray and the desired properties of the tray in the molten or softened ("warm") and solid ("cool") states. The warm state is characterized by appreciable mass flow of the thermoplastic material under moderate (hand) pressure at some temperature between body temperature (about 38°C) and the maximum temperature that comfortably can be withstood by oral tissues. This maximum temperature is generally thought to be about 75°C, although a maximum of about 65°C is preferred. The cool state is characterized by sufficient strength and stiffness to permit an acceptably accurate dental impression to be made in the tray, and by minimal apparent mass flow of the thermoplastic material under moderate pressure at temperatures below 38°C.

The warm and cool state properties permit the tray to be heated to a moderate temperature, manually shaped in the mouth while warm to conform to the shape of hard and soft oral tissue, and cooled within the mouth to form a substantially rigid custom impression tray.

Representative thermoplastic materials include polyesters and polyurethanes such as those described in U.S. Pat. Nos. 3,382,202, 4,059,715, 4,182,829, 4,327,013, 4,361,538, 4,552,906 and 4,569,342; copolymers such as those described in U.S. Pat. Nos. 4,659,786, 4,740,245 and 4,768,951; and ethylene-vinyl acetate copolymers such as those described in European Published Pat. Appl. No. 0 359 135 and Kokai No. Sho 63[1988]-96536. The thermoplastic material preferably is a homopolymer or copolymer of epsilon-caprolactone. The polycaprolactone optionally can contain property-modifying or cross-linkable functional groups (for example hydroxyl, acrylate, methacrylate, epoxy, isocyanato or vinyl groups) if desired. Blends of polycaprolactones can also be employed.

Preferred polycaprolactones have the formula: 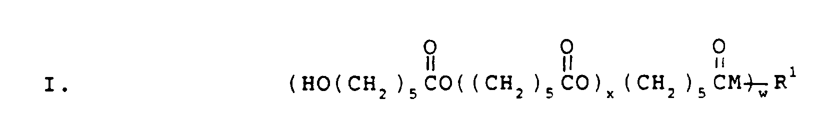
where R¹ is hydrogen or an aromatic or a straight chain or branched aliphatic backbone, which can optionally contain one or more non-interfering substituents such as hydroxyl or amine groups, w is 1 if R¹ is hydrogen, and w otherwise has an average value of about 1 to about 4, M is oxygen or -NR²- where R² is hydrogen or a non-interfering aromatic or aliphatic group, and the product of w times x is greater than about 35.

Preferred commercially available polycaprolactone polymers include "TONE P-700" and "TONE P-767" (40,000 molecular weight) and "TONE P-300" (10,000 molecular weight) polycaprolactone from Union Carbide Corp., and the "CAPA" polycaprolactones "630" (30,000 molecular weight), "640" (40,000 molecular weight), "650" (50,000 molecular weight), and "656" (56,000 molecular weight) from Interox.

The thermoplastic material preferably contains at least one ethylenically unsaturated monomer, oligomer, or polymer capable of undergoing addition polymerization, together with a suitable initiator (e.g., a photoinitiator). These can be crosslinked to impart shape memory to the thermoplastic material, thereby converting it to a semi-thermoplastic substance.

The thermoplastic material can contain a wide variety of adjuvants depending upon the desired end use. Suitable adjuvants include solvents, diluents, plasticizers, pigments, dyes, inorganic or organic reinforcing or extending fillers, thixotropic agents, indicators, inhibitors, stabilizers, UV absorbers, medicaments (e.g., leachable fluorides), biocides and the like. The thermoplastic material preferably contains one or more fillers that increase the material's warm state modulus. Preferred fillers have a fibrous, platy or finely-divided particulate form. For fibrous or platy fillers, the filler preferably has an aspect ratio greater than about 3:1, more preferably greater than about 5:1, and most preferably greater than 10:1. For particulate fillers, the particles preferably have an average particle diameter less than about 100 micrometers, more preferably less than about 10 micrometers, and most preferably less than about 1 micrometer. Particularly preferred fillers include glass fibers, clay, mica, alumina, submicron silica (for example, fumed, precipitated or colloidal silica) and finely-divided calcium carbonate. The fillers can be untreated or treated with a suitable coupling agent (for example, a silane).

The types and amounts of ingredients in the thermoplastic material usually will be empirically selected. The thermoplastic material preferably should remain substantially homogeneous (that is, it should not undergo macroscopic phase separation or filler sedimentation). Subject to the effects of any imprinted shape memory, the tray preferably should retain its desired physical properties even if repeatedly cycled between the warm and cool states. Thus the selection of ingredients can be guided in part by the desire to preserve homogeneity and thermal reversibility. As a further guide, the preferred amounts of thermoplastic material, polymerizable resin, initiator and filler for dental impressioning are as follows:

| Ingredient | Preferred Weight % | Most Preferred Weight % |
|---|---|---|
| Thermoplastic material | up to 90 | 80 to 40 |
| Polymerizable resin | 0 to 50 | 0 to 35 |
| Initiator | 0 to 10 | 0 to 5 |
| Filler | 0 to 70 | 20 to 60 |

The ingredients can be blended by hand or by mechanical mixing. The ingredients preferably are warmed sufficiently to melt the thermoplastic material, but if desired can be mixed at lower temperatures. Any suitable mixing device can be used, including kettles equipped with a mechanical stirrer, extruders, rubber mills, and the like.

A variety of woven, nonwoven or perforated sheetlike materials can be used to form the scrim layer. Suitable woven materials include cloth or gauze formed of natural or synthetic fibers such as cotton, polyester (e.g., "DACRON" fibers, and "SONTARA" fabrics such as polyester grades 8000, 8027 and 8100, E. I. duPont de Nemours & Co.), polyethylene, polypropylene, polyurethane, polyamide (e.g., "NYLON" fiber), polyaramide (e.g., "KEVLAR" fiber) and glass (e.g., "FIBERGLAS" fiber). The scrim can be a conventional weave or a nonconventional weave such as either the "hook" or "loop" fabrics used in hook and loop fasteners. Suitable nonwoven materials include felt and randomly-laid polyester (e.g., "BONAR" fabrics such as "ULTRALOFT" powder-bonded polyester grades 60B-9125, 60B-9628 and 60B-9928, Bonar Fabrics Corporation). Suitable perforated sheetlike materials include fine pitch polypropylene net. The scrim preferably should bond well to both the thermoplastic material and to the intended impression material, be free of objectionable taste or odor, and be safe for use in the mouth. The scrim also preferably has a melting temperature above the melting or softening temperature of the thermoplastic material. Woven or perforated sheetlike scrim materials preferably have a mesh size and surface topography sufficient to provide the desired degree of adhesion between the tray and the impression material.

If desired, the scrim can be treated with an appropriate primer or coupling agent to aid adhesion to the thermoplastic material or to the impression material. The tray side of the scrim can be coated with a suitable thermoplastic coating or film capable of adhering the scrim to the tray. The scrim can also be printed with instructions for the user or imprinted with a suitable trademark.

The tray is conveniently assembled by first forming the thermoplastic material into a thin web. The web can be shaped in a variety of ways including extrusion, injection molding, or coating using a suitable coating knife and rollers. If desired, the thermoplastic material can be extruded or cast in multiple layers (for example, coplanar layers or layers arranged in core-shell fashion) in which each layer has a selected melting temperature, viscosity, modulus, stickiness, or other desired physical properties.

If desired, the thermoplastic material can be cast onto the scrim. However, it is usually more convenient to press the scrim into a major surface of the thermoplastic web sheet. Heated nip rolls can be used if desired to control caliper and ensure a good bond between the thermoplastic material and the scrim.

The resulting scrim-covered assembly can be formed into trays using conventional techniques. Vacuum molding is particularly useful. Preferably one or more spacers are fastened to the scrim. The spacers can be placed atop the scrim or immediately beneath it. These aid in controlling the amount of impression material that will be loaded into the tray, and in preventing unduly thin impressions which may perforate or tear upon removal from the mouth. Preferably one or more of the spacers are thermoplastic, e.g., of the same thermoplastic material from which the tray shell is made. By heating a thermoplastic spacer or the scrim prior to assembly, a firm bond between spacer and scrim will readily be achieved.

The completed trays can be converted by the user from the cool state to the warm state with a variety of energy sources. The tray can be immersed in a heated bath containing a suitable inert liquid (for example, water or a fluorochemical fluid) that will not dissolve or swell the tray in either its cool or warm states. The tray can also be softened using heat sources such as a hot air gun, hot plate, conventional oven, infrared heater or microwave oven. The tray can be encased in a plastic pouch or other container which is in turn heated (e.g., electrically), or subjected to one or more of the above-mentioned heating methods.

Transforming the tray from a warm state to a cool state requires loss of thermal energy and can be carried out using a variety of cooling techniques. Cooling can take place under ambient conditions in the presence of air only. Cooling can be expedited using forced air, cold water, ice, or heat sinks such as chilled "cold packs" or flexible pouches containing low boiling inert liquids. Of particular interest for dental applications are chilled cold packs in flexible pouches that have been preshaped to match the contours of the tray. For example, flexible pouches containing a chilled coolant can be fabricated in the shape of a full arch or quadrant and placed intraorally in contact with the warm tray.

Shape memory can be imparted to trays containing a free-radically polymerizable resin and initiator by allowing or causing the resin to harden (for example, by exposing a composition containing a photoinitiator to a suitable light source). Polymerization can take place before or after the warmed, softened tray is allowed to cool, although carrying out polymerization after cooling will tend to lengthen polymerization times. After polymerization, the tray preferably retains sufficient elasticity to permit it to be removed from undercut surfaces such as the undercuts typically found in the oral cavity. The polymerized, cooled tray will exhibit shape memory. Accordingly, it will be more resistant to heat and handling stresses than trays made from the thermoplastic material alone. If the tray is accidentally distorted, it can be returned to its custom shape by reheating it in a relaxed condition. For example, the tray can be immersed in a hot water bath and removed for cooling after the custom shape has reappeared. While still in the warm state, it will remain pliable, and accordingly will exhibit semi-thermoplasticity. This permits the custom shape to be adjusted if desired.

If desired, a scrim-lined tray of the invention can be filled with a conventional dental impression material (for example, a polyether or an addition-cured silicone). By shaping the tray in the mouth before (or if desired, after) it is filled with impression material, the tray volume and required amount of impression material will be minimized.

The following examples are offered to aid in understanding the invention and are not to be construed as limiting its scope. Unless otherwise indicated, all parts and percentages are by weight.

### EXAMPLE 1

A filled thermoplastic custom tray composition was prepared by combining 22.5 parts "TONE P-767" 40,000 molecular weight polycaprolactone (Union Carbide Corp.), 7.5 parts "SR-9505" urethane diacrylate oligomer (Sartomer Corp.), 1.2 parts "EBERCRYL 830" polyester hexacrylate (Radcure Specialties, Inc.), 45 parts "VICRON" calcium carbonate (Pfizer Corp.), 10 parts "SIPERNAT D-11" treated precipitated silica (North American Silica Co.), 5 parts "ULTRASIL VN 3 SP" untreated precipitated silica (North American Silica Co.), 4 parts "1156" chopped glass fibers (PPG), and 0.75 parts each camphorquinone ("CPQ") and p-ethyldimethylaminobenzoate ("EDMAB"). The ingredients were stirred in a warm vessel at about 100°C until homogeneous. The resulting mixture was cast atop polyester film into a 2.5mm thick layer into which was pressed a web of "SONTARA 8000" nonwoven material (E.I. DuPont de Nemours & Co.). The warm assembly was cut into generally U-shaped pieces using a "cookie cutter" style cutting implement. Each piece was squeezed while still warm between two halves of a silicone mold to form an arch-shaped, nonwoven scrim-lined customizable tray. The tray could be reheated and shaped while warm to conform to a patient's dentition. When irradiated for two minutes in a "STAR-CURE" curing chamber (Star X-ray, Inc.), a tray with shape memory was obtained. The tray can also be photocured by setting it under a conventional dental operatory light (for example, a "RITTER STAR LIGHT", Sybron Corp.) for about two minutes per side.

### EXAMPLE 2

A thermoplastic custom tray was prepared by combining 36.7 parts "TONE P-767" polycaprolactone, 12 parts "SR-9505" urethane diacrylate oligomer, 2 parts "EBERCRYL 830" polyester hexacrylate, 20 parts "3075" chopped glass fibers (PPG), 11.7 parts "QUSO WR55" hydrophobic silica (North American Silica Co.), 16.6 parts "ULTRASIL VN 3 SP" untreated precipitated silica, 0.9 parts EDMAB, and 0.23 parts CPQ. The ingredients were stirred in a warm vessel at 100°C until homogeneous. The resulting blend was molded in the shape of a full arch tray using a conventional injection molding apparatus. The interior of the completed tray was softened by heating it under an infrared lamp. A sheet of "SONTARA 8027" polyester fabric was pressed into the heated tray surface, and the tray was allowed to cool.

The tray was warmed in a 60°C water bath, placed in Dr. Oxman's mouth, shaped to conform to his maxillary arch, and allowed to cool until hardened. The resulting custom impression dental tray was removed and then irradiated by placing it at the focal point of a "RITTER STAR LIGHT" for 2.5 minutes per side. The interior of the tray was coated with a thin layer of "IMPRINT" single phase vinyl siloxane impression material (3M), then reinserted in Dr. Oxman's mouth, pressed against his maxillary arch, and allowed to set for about 4 minutes. The resulting impression was readily removed from his mouth without tearing or delamination of the impression material. The cured siloxane impression material in the completed tray had a total volume less than 15 ml. A typical impression prepared in a stock tray would have required about 30 ml of impression material. Thus a vinyl siloxane material savings of over 50% was realized, and the impression was completed without resorting to the customary solvent-based tray adhesive. Because vinyl siloxane impression materials are very expensive (owing in part to the presence of a platinum catalyst and other expensive ingredients), the reduced impression material requirement was particularly beneficial.

### EXAMPLE 3

In a controlled confidential evaluation, impression trays like those of EXAMPLE 2 were used to prepare over 100 single or multiple unit crowns and single or multiple unit bridges in human subjects. No remakes were required. Despite the absence of a tray adhesive, adhesion of the impression materials to the trays was regarded as "excellent" by the 14 dentists carrying out the evaluation.

### EXAMPLES 4-6

Impression trays like those of EXAMPLE 2 were used to prepare custom dental trays on a "TYPODONT" lower arch model (Columbia Dentoform, Inc.). Impressions were then prepared using the following impression materials:

| Ex. No. | Impression Material | Manufacturer |
|---|---|---|
| 4 | "XANTOPREN PLUS" light body condensation-cured silicone | Bayer, Inc. |
| 5 | "IMPREGUM F" regular body polyether | Espe-Premier, Inc. |
| 6 | "PERMLASTIC" regular body polysulfide | Kerr Division of Sybron, Inc. |

The impression materials of EXAMPLES 4 and 5 were cured in air at room temperature. The impression material of EXAMPLE 6 was cured in a 37°C water bath. The manufacturer-recommended cure time was used for each impression material. In each instance an excellent bond was formed between the impression material and tray, without employing a tray adhesive. The required volume of impression material was approximately one-half that needed for an impression in a conventional stock tray.

Although this invention has been described using certain illustrative examples, it should be understood that the invention is not limited to the specific exemplary embodiments shown in this specification.

## Claims

1. A scrim-lined thermoplastic dental impression tray (5,11,21), comprising a flat or curved shell (1a,13,23) of a thermoplastic material comprising a polymer selected from the group consisting of polyesters, polyurethanes, copolymers of ethylene-vinyl acetate, and homopolymers and copolymers of epsilon-caprolactone that is solid at 38°C and has a melting or softening point that comfortably can be withstood by oral tissues, for example at some temperature between body temperature and about 75°C, the shell having on at least one of its major surfaces a firmly-adhered, exposed sheet (3a,15,31) of natural or synthetic woven or nonwoven fabric.

2. A tray according to claim 1, wherein the sheet (3a,15,31) comprises cotton, polyester, polyethylene, polypropylene, polyurethane, polyamide, or polyaramide.

3. A tray according to claim 1, wherein the sheet (3a,15,31) comprises woven natural or synthetic fibre.

4. A tray according to claim 1, wherein the sheet (3a,15,31) comprises a hook or loop fabric or polypropylene netting.

5. A tray according to claim 1, wherein the sheet (3a,15,31) comprises a nonwoven fabric.

6. A tray according to claim 5, wherein the nonwoven fabric comprises a randomly-laid polyester.

7. A tray according to claim 1, wherein a coating or film of thermoplastic (23) is interposed between the sheet (31) and the tray (25).

8. A tray according to claim 1, wherein one or more spacers lie atop or beneath the sheet.

9. A tray according to claim 8, wherein at least one spacer button comprises a thermoplastic material.

10. A tray according to claim 1, wherein said thermoplastic material further comprises at least one ethylenically unsaturated monomer, oligomer, or polymer capable of undergoing addition polymerization, and an initiator.

11. A tray according to claim 10, wherein said ethylenically unsaturated monomer, oligomer, or polymer comprises an acrylate or methacrylate group and said initiator comprises a photoinitiator.

12. A tray according to claim 11, wherein said sheet (3a,15,31) provides an effective barrier between said acrylate or methacrylate functional monomer, oligomer, or polymer and a siloxane impression material placed against said sheet.

13. A method for preparing a custom impression tray without the need for a tray adhesive, comprising the steps of (a) softening a scrim-lined thermoplastic dental impression tray (5,11,21) comprising a flat or curved shell of a thermoplastic material comprising a polymer selected from the group consisting of polyesters, polyurethanes, copolymers of ethylene-vinyl acetate, and homopolymers and copolymers of epsilon-caprolactone that is solid at 38°C and has a melting or softening point that comfortably can be withstood by oral tissues, for example at some temperature between body temperature and about 75°C, the shell having on at least one of its major surfaces a firmly-adhered, exposed sheet (3a,15,31) of natural or synthetic woven or nonwoven fabric, by heating the tray above the melting or softening point of the thermoplastic material, (b) shaping the tray, (c) cooling the tray so that it resolidifies, and (d) at least partially filling the tray with an impression material.

14. A method according to claim 13, wherein the sheet (3a,15,31) comprises cotton, polyester, polyethylene, polypropylene, polyurethane, polyamide, or polyaramide.

15. A method according to claim 13, wherein the sheet (3a,15,31) comprises woven natural or synthetic fibre.

16. A method according to claim 13, wherein the sheet (3a,15,31) comprises a nonwoven fabric.

17. A method according to claim 16, wherein the nonwoven fabric (3a,15,31) comprises a randomly-laid polyester.

18. A method according to claim 13, wherein the tray comprises a full arch or quadrant tray.

19. A method according to claim 13, wherein the tray comprises a double bite tray.

20. A method according to claim 13, wherein the shell comprises a polycaprolactone.

21. A method according to claim 13, wherein said thermoplastic material further comprises at least one ethylenically unsaturated monomer, oligomer, or polymer capable of undergoing addition polymerization, and an initiator.

## Patentansprüche

1. Mit einer Verstärkungseinlage versehener thermoplastischer Dentalabdrucklöffel (5,11,21) mit einer flachen oder gekrümmten Schale (1a,13,23) aus thermoplastischem Material, das ein Polymer aufweist, welches aus der Gruppe Polyester, Polyurethane, Kopolymere von Ethylen-Vinyl-Azetat, und Homopolymere und Kopolymere von ε-Caprolacton gewählt ist, und das bei 38°C fest ist und einen Schmelz- oder Erweichungspunkt aufweist, der von der Oralgewebe-Temperatur unerreicht bleibt und z.B. bei einer Temperatur zwischen der Körpertemperatur und ungefähr 75°C liegt, wobei die Schale mindestens an einer ihrer Hauptflächen eine fest anhaftende, freiliegende Lage (3a,15,31) aus natürlichem oder synthetischen gewebtem oder vliesartigen Material aufweist.

2. Löffel nach Anspruch 1, bei dem die Lage (3a,15,31) Baumwolle, Polyester, Polyethylen, Polypropylen, Polyurethan, Polyamid oder Polyaramid aufweist.

3. Löffel nach Anspruch 1, bei dem die Lage (3a,15,31) gewebte Natur- oder Kunstfaser aufweist.

4. Löffel nach Anspruch 1, bei dem die Lage (3a,15,31) einen Maschen- oder Loop- Stoff oder ein Polypropylen-Gewebe aufweist.

5. Löffel nach Anspruch 1, bei dem die Lage (3a,15,31) einen Vliesstoff aufweist.

6. Löffel nach Anspruch 5, bei dem der Vliesstoff ein willkürlich gelegtes Polyester aufweist.

7. Löffel nach Anspruch 1, bei dem zwischen der Lage (31) und dem Löffel (21) eine Beschichtung oder ein Film aus thermoplastischem Material (23) angeordnet ist.

8. Löffel nach Anspruch 1, bei dem ein oder mehrere Abstandshalter oberhalb oder unterhalb der Lage angeordnet sind.

9. Löffel nach Anspruch 8, bei dem mindestens ein Abstandshalter-Knopf ein thermoplastisches Material enthält.

10. Löffel nach Anspruch 1, bei dem das thermoplastische Material ferner mindestens ein ethylenisch ungesättigtes Monomer, Oligomer oder Polymer, das einer Additionspolymerisation unterzogen werden kann, und einen Initiator aufweist.

11. Löffel nach Anspruch 10, bei dem das ethylenisch ungesättigte Monomer, Oligomer oder Polymer eine Akrylat- oder Methakrylat-Gruppe und der Initiator einen Photoinitiator aufweist.

12. Löffel nach Anspruch 11, bei dem die Lage (3a,15,31) eine wirksame Barriere zwischen dem Akrylat- oder Methakrylat-Funktional-Monomer, -Oligomer oder -Polymer und einem gegen die Lage angelegten Siloxan-Abdruckmaterial schafft.

13. Verfahren zum Vorbereiten eines patientenspezifischen Abdrucklöffels ohne die Notwendigkeit eines Löffel-Klebers, mit dem folgenden Verfahrensschritten: (a) durch Erwärmen des Löffels über den Schmelz- oder Erweichungspunkt des thermoplastische Materials, Erweichen eines mit einer Verstärkungseinlage versehenen thermoplastischen Dentalabdrucklöffels (5,11,21) mit einer flachen oder gekrümmten Schale aus thermoplastischem Material, das ein Polymer aufweist, welches aus der Gruppe Polyester, Polyurethane, Kopolymere von Ethylen-Vinyl-Azetat, und Homopolymere und Kopolymere von ε-Caprolacton gewählt ist, und das bei 38°C fest ist und einen Schmelz- oder Erweichungspunkt aufweist, der von der Oralgewebe-Temperatur unerreicht bleibt und z.B. bei einer Temperatur zwischen der Körpertemperatur und ungefähr 75°C liegt, wobei die Schale mindestens an einer ihrer Hauptflächen eine fest anhaftende, freiliegende Lage (3a,15,31) aus natürlichem oder synthetischen gewebtem oder vliesartigen Material aufweist, (b) Formen des Löffels, (c) Kühlen des Löffels zu dessen Wiederaushärtung, und (d) mindestens teilweises Füllen des Löffels mit einem Abdruckmaterial.

14. Verfahren nach Anspruch 13, bei dem die Lage (3a,15,31) Baumwolle, Polyester, Polyethylen, Polypropylen, Polyurethan, Polyamid oder Polyaramid aufweist.

15. Verfahren nach Anspruch 13, bei dem die Lage (3a,15,31) gewebte Natur- oder Kunstfaser aufweist.

16. Verfahren nach Anspruch 13, bei dem die Lage (3a,15,31) einen Vliesstoff aufweist.

17. Verfahren nach Anspruch 16, bei dem der Vliesstoff (3a, 15,31) ein willkürlich gelegtes Polyester aufweist.

18. Verfahren nach Anspruch 13, bei dem der Löffel ein Vollbogen- oder ein Qudranten-Löffel ist.

19. Verfahren nach Anspruch 13, bei dem der Löffel ein Doppelbißlöffel ist.

20. Verfahren nach Anspruch 13, bei dem die Schale ein Polycaprolacton aufweist.

21. Verfahren nach Anspruch 13, bei dem das thermoplastische Material ferner mindestens ein ethylenisch ungesättigtes Monomer, Oligomer oder Polymer, das einer Additionspolymerisation unterzogen werden kann, und einen Initiator aufweist.

## Revendications

1. Porte-empreinte dentaire (5, 11, 21) en matériau thermoplastique revêtu de mousseline, comprenant un moule (1a, 13, 23) disposé à plat ou incurvé, formé d'un matériau thermoplastique comprenant un polymère choisi dans l'ensemble constitué par les polyesters, les polyuréthanes, les copolymères d'éthylène-acétate de vinyle et les homopolymères et copolymères d'epsilon-caprolactone, qui est solide à 38°C et a un point de fusion ou un point de ramollissement qui peut être supporté aisément par les tissus buccaux, par exemple à une température comprise entre la température du corps et environ 75°C, le moule comprenant, au moins sur une de ses surfaces principales, une feuille (3a, 15, 31) exposée et solidement collée, constituée d'un tissé ou d'un non-tissé naturel ou synthétique.

2. Porte-empreinte selon la revendication 1, dans lequel la feuille (3a, 15, 31) est constituée de coton, de polyester, de polyéthylène, de polypropylène, de polyuréthane, de polyamide ou de polyaramide.

3. Porte-empreinte selon la revendication 1, dans lequel la feuille (3a, 15, 31) est constituée de fibres naturelles ou synthétiques tissées.

4. Porte-empreinte selon la revendication 1, dans lequel la feuille (3a, 15, 31) est constituée d'un tissu crocheté ou d'un tissu bouclé ou d'un fileté en polypropylène.

5. Porte-empreinte selon la revendication 1, dans lequel la feuille (3a, 15, 31) est constituée d'un non-tissé.

6. Porte-empreinte selon la revendication 5, dans lequel le non-tissé comprend un polyester posé de façon aléatoire.

7. Porte-empreinte selon la revendication 1, dans lequel un revêtement ou un film de matériau thermoplastique (23) est interposé entre la feuille (31) et le porte-empreinte (25).

8. Porte-empreinte selon la revendication 1, dans lequel un ou plusieurs écarteurs sont situés au-dessus ou au-dessous de la feuille.

9. Porte-empreinte selon la revendication 8, dans lequel au moins un bouton d'espacement est constitué d'un matériau thermoplastique.

10. Porte-empreinte selon la revendication 1, dans lequel ledit matériau thermoplastique comprend, en outre, au moins un monomère, un oligomère ou un polymère éthyléniquement insaturé, qui est capable de subir une polymérisation par addition, et un initiateur.

11. Porte-empreinte selon la revendication 10, dans lequel ledit monomère, oligomère ou polymère éthyléniquement insaturé est constitué par un groupe acrylate ou un groupe méthacrylate, et ledit initiateur est constitué par un photo-initiateur.

12. Porte-empreinte selon la revendication 11, dans lequel ladite feuille (3a, 15, 31) fournit une barrière efficace entre ledit monomère, oligomère ou polymère à fonctionnalité acrylate ou méthacrylate et un matériau d'empreinte à base de siloxane qui est placé contre ladite feuille.

13. Procédé de préparation d'un porte-empreinte à façon sans la nécessité d'un adhésif pour porte-empreinte, comprenant les étapes consistant à (a) ramollir un porte-empreinte dentaire (5, 11, 21) en matériau thermoplastique revêtu de mousseline, comprenant un moule disposé à plat ou incurvé, formé d'un matériau thermoplastique comprenant un polymère choisi dans l'ensemble constitué par les polyesters, les polyuréthanes, les copolymères d'éthylène-acétate de vinyle et les homopolymères et copolymères d'epsilon-caprolactone, qui est solide à 38°C et a un point de fusion ou un point de ramollissement qui peut être supporté aisément par les tissus buccaux, par exemple à une température comprise entre la température du corps et environ 75°C, le moule comprenant, au moins sur une de ses surfaces principales, une feuille (3a, 15, 31) exposée et solidement collée, constituée d'un tisse ou d'un non-tissé naturel ou synthétique, en chauffant le porte-empreinte à une température située au-dessus du point de fusion ou du point de ramollissement du matériau thermoplastique, (b) façonner le porte-empreinte, (c) refroidir le porte-empreinte de manière qu'il se solidifie de nouveau et (d) remplir le porte-empreinte au moins partiellement avec un matériau pour empreinte.

14. Procédé selon la revendication 13, dans lequel la feuille (3a, 15, 31) est constituée de coton, de polyester, de polyéthylène, de polypropylène, de polyuréthane, de polyamide ou de polyaramide.

15. Procédé selon la revendication 13, dans lequel la feuille (3a, 15, 31) est constituée de fibres naturelles ou synthétiques tissées.

16. Procédé selon la revendication 13, dans lequel la feuille (3a, 15, 31) est constituée d'un non-tissé.

17. Procédé selon la revendication 16, dans lequel le non-tissé (3a, 15, 31) comprend un polyester posé de façon aléatoire.

18. Procédé selon la revendication 13, dans lequel le porte-empreinte est constitué par un porte-empreinte en arc plein ou en quart de cercle.

19. Procédé selon la revendication 13, dans lequel le porte-empreinte est constitué par un porte-empreinte à double occlusion.

20. Procédé selon la revendication 13, dans lequel le moule comprend une polycaprolactone.

21. Procédé selon la revendication 13, dans lequel ledit matériau thermoplastique comprend, en outre, au moins un monomère, un oligomère ou un polymère éthyléniquement insaturé, qui est capable de subir une polymérisation par addition, et un initiateur.
